# EUROPEAN PATENT SPECIFICATION

(11) **EP 0 676 183 B1**
(45) Date of publication and mention of the grant of the patent: **12.07.2000**
(21) Application number: 95302334.8
(22) Date of filing: 07.04.1995
(51) Int. Cl.: A61F 13/02

(54) **Conformable adhesive bandages**
Konformierbare Klebverbände
Bandages adhésifs conformes

(30) Priority: 08.04.1994 US 225171; 11.04.1994 US 226134
(43) Date of publication of application: 11.10.1995
(73) Proprietor: JOHNSON & JOHNSON CONSUMER PRODUCTS, INC., Skillman, New Jersey 08558-9418 (US)
(72) Inventor: Finley, George B., Yardley, PA 19067 (US); Catalano, Marlon S., Edison, NJ 08820 (US); Davis, Ian, Annadale, NJ 08801 (US)
(74) Representative: Fisher, Adrian John

(56) References cited:
- EP-A- 0 099 748
- EP-A- 0 249 694
- EP-A- 0 541 251
- FR-A- 2 076 050
- GB-A- 2 228 682
- US-A- 3 025 854
- US-A- 4 341 209
- US-A- 4 484 574

## Description

### 1. Field Of The Invention

This invention relates to disposable bandages and dressings for minor and major skin wounds, irritations and abrasions. More particularly, this invention relates to disposable bandages which are extremely flexible and conformable to the wearer's skin. They permit the wearer to experience comfort and the bandage to maintain adhesion to the skin using relatively low adhesive weights and/or less aggressive adhesives.

### 2. Background Of The Invention

For many years, disposable bandages have been sold for the purpose of assisting in the treatment of cuts and bruises. They function to cover and protect the wound while it heals. Such disposable bandages have contained a backing material, which serves as a substrate and protective covering for the wound, a pad, which overlays the cut or bruise, and an adhesive composition, which is deposited on the backing material. The adhesive composition adheres the pad to the backing and, where exposed, adheres the backing to the skin surrounding the wound area. However, there are several problems inherent in the use of such bandages. First, the wearer often has abrasions in areas of the skin which move frequently, such the fingers, elbow and knee joints. This subjects the bandage to forces which tend to deform the bandage. This deformation often results in delamination of the bandage from the skin. Second, in efforts to combat this delamination, bandages have been made with fairly aggressive adhesive compositions. These tend to irritate sensitive skin and may injure skin when they are removed from the wearer.

Various changes and improvements have been made over the years in the commercial embodiments of disposable bandage products. For example, backing materials have been made from vinyl film, flexible fabric and even foam in attempts to create bandages which conform more readily to the skin and movements of the wearer. However, attempts to date have not provided consumers with bandages which conform readily with movement while remaining tenaciously adhered to the skin.

For example, U.S. patent No. 3,025,854 (Scholl, 1962) describes a bandage having a film backing and a foam, rather than a gauze, pad attached. However, this construction does not necessarily provide appropriate ventilation to the wound.

In U.S. Patent No. 3,113, 568 (Robins, 1963), a "styptic" bandage containing an absorptive pad with a closely-knit, reticulated barrier was described. The reticulated barrier was intended to aid in the stanching of blood from the wound and in the formation of a clot to promote healing. The patent suggests that such a barrier may be composed of open-celled, polyurethane foam.

U.S. Patent No. 3,949,742 (Nowakowski, 1976) describes a medical dressing constructed of a transparent, thin and flexible laminate of a thin layer of non-porous segmented polyurethane backing cohesively secured to a thin layer of thrombogenic reticulated foam. The backing layer is intended to be non-porous and extremely thin.

U.S. Patent No. 4,733,659 (Edenbaum et al., 1988) relates to a disposable bandage prepared from a single sheet of liquid permeable, flexible thermoplastic hydrophilic foam. The foam was laminated with a porous pressure-sensitive adhesive. The foam laminate was then compressed in its entirety, but for the portion intended to contact the wound.

U.S. Patent No. 4,730,611 (Lamb, 1988) relates to a dressing device composed of a foamed polyurethane pad which has a hydrophilic side for contact with the wound and a hydrophobic side for facing away from the wound. The pad was positioned on a porous non-woven fibrous sheet which was larger than the pad. It had an adhesive on the side contacting the hydrophobic side of the pad so that the device presented the hydrophilic side and the adhesive for use in contact with the wound. A non-sticking removable cover was placed over the adhesive side of the sheet to prevent early exposure of the pad and the adhesive. This device had the disadvantage of having an extremely complex construction, not readily usable in connection with minor cuts and abrasions.

U.S. Patent No. 4,341,209 (Schaar, 1982) describes a pressure sensitive adhesive bandage including a low density polyethylene closed cell foam backing sheet with a glossy, smoothly pebbled, low friction, non-adherent face surface and a coating-adherent rear surface having a pressure-sensitive adhesive which is adhered to an absorbent pad. The polyethylene foam was intended to replace vinyl film, which normally contains plasticizers. It was intended to have the same material properties as vinyl.

U.S. Patent No. 4,377,159 (Hansen, 1983) describes a pressure bandage having a substantially triangular or rectangular prism-shaped section of compressible, resilient material which has been adhered to a carrier tape. the tape would not provide additional conformability.

Hydrogel bandages, such as those described in U.S. patent No. 5,160,328 (Cartmell et al., 1992) can contain a polyurethane hydrogel material, but again, does not improve upon the conformability of the backing used in such bandages.

U.S. Patent No. 5,203,764 (Libbey et al., 1993) relates to the use of an open-celled foam pad prepared from adc foam sheet impregnated with a water curable isocyanate functional polyurethane prepolymer resin to dress a wound. The pad can also function as an orthotic pad.

U.S. patent No. 5,264,218 (Rogozinski, 1993) relates to a dressing for wounds or dermal ulcers including a semi-permeable, transparent polyurethane and a plurality of concentric polyethylene foam rings or disks.

EP-A-054251, EP-A-0 099 748, GB-A-2 228 682 and FR-A-2 076 050 an disclose disposable adhesive bandages comprising a relatively conformable backing material, an adhesive layer and an absorbent pad.

None of the prior art patents, however, suggests or describes a disposable adhesive bandage product which is highly conformable yet maintains tenacious adhesive attachment to the skin of the wearer, even at low adhesive weights.

### Summary Of The Invention

This invention relates to a highly conformable, disposable adhesive bandage. More particularly, it relates to a highly conformable, disposable adhesive bandage composed of a conformable backing material, a layer of adhesive and an absorbent pad.

Preferably, the backing material is composed of a foam or a fabric substrate which is highly conformable to the skin of a wearer. Conformability can be measured in a variety of way, in particular by the stress/strain modulus. The stress/strain modulus of the products of this invention is relatively low, i.e., the products require a force of 36 to 125 g/cm (0.2 to 0.7 lb/in) to produce a 20% strain, and a force of 214 to 321 g/cm (1.2 to 1.8 lb/in) to produce a 50% strain. Preferably the products require a force of 89 g/cm (0.5 lb/in) for 20% strain and 268 g/cm (1.5 lb/in) for 50% strain.

The adhesive compositions useful in the products of this invention are preferably pressure-sensitive adhesives that are clinically proven to be biocompatible, or compatible with the skin of the wearer. A smaller loading of adhesive composition is possible in the products of this invention than was previously needed in order to maintain adhesion with the skin of the wearer. Furthermore, less aggressive adhesives may be used. This is because the products of this invention tend to bend and move readily with the skin of the wearer and do not exert forces opposing such movement which would ordinarily pull the bandage away from the skin. Advantageously, this permits the products of this invention to be less irritating to the wearer's skin and prevents skin damage due to removal.

The absorbent pad of the products of this invention are, preferably, composed of a wound-release portion intended to contact the wound itself and an absorbent portion into which the wound exudates can be absorbed and contained.

### Description Of The Figures

Figure 1 is a bottom plan view of a disposable bandage made in accordance with this invention.

Figure 2 is a bottom plan view of a disposable bandage made in accordance with this invention.

Figure 3 is a bottom plan view of a disposable bandage made in accordance with this invention.

Figure 4 is a chart illustrating data obtained from a test of several bandages measuring maximum force vs. strain.

Figure 5 is a chart illustrating data obtained from a test of several bandages measuring recovered energy vs. strain.

Figure 6 is a chart illustrating data obtained from a test of several bandages measuring tensile breaking strength.

Figure 7 is a chart illustrating data obtained from a test of several bandages measuring maximum elongation.

### Detailed Description Of The Preferred Embodiments

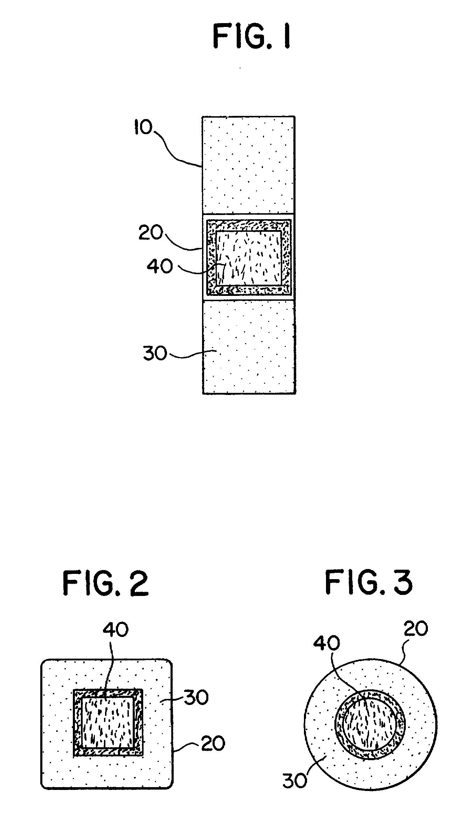

The products of this invention contain at least three elements: the backing material, which protects the wound-covering portion of the bandages of this invention; the wound-covering portion of the products, which contacts the wound and provides a reservoir for the wound exudate; and the adhesive composition, which holds the products in place around the wound. Embodiments of the bandages of this invention may be found in Figures 1, 2 and 3. In Figure 1, bandage 10 is a disposable, removably adherable bandage according to one embodiment of this invention. Bandage 10 is composed of a backing material 20, an adhesive layer 30 and an absorbent pad 40. Backing material 20 is preferably composed of a polymeric foam or a fabric material which lends the appropriate conformability characteristics to the bandages of this invention. More preferably, backing material 20 is composed of a closed-cell polyvinyl chloride foam or film, polyethylene foam or film, or a polyurethane foam or film. Nonwovens having the appropriate characteristics, such as meltblowns, such as those containing Kraton fibers, or spunbond, all made from natural or synthetic cellulose fibers or other fibers known to those of ordinary skill in the art, may be useful in the products of this invention. The backing material should have stretch and recovery properties that are the same or similar in all directions, so as to provide conformability and strength in all directions during wear.

Backing material 20 is most preferably plasticized polyvinyl chloride foam having approximately 0.48 g/cm³ (30 lb/ft³) density and, preferably, about 0.5mm (20 mils) thick. The backing material of the products of this invention must possess the characteristics of flexibility and resilience so as to produce bandages having the desired characteristics according to this invention. Preferably, the density of polyvinyl chloride foams useful in the products of this invention are between about 0.4 and about 0.56 g/cm³ (about 25 and about 35 lbs/ft³) and between about 0.38 and about 0.63 mm (about 15 and about 25 mils) thick. The thickness of the foam in comparison with prior art backing materials, such as vinyl film, also provides a cushioning effect to protect the wound from external forces. If a hydrophobic or moisture-impermeable foam is used, the foam should be punctured or perforated with small holes in order to provide means for moisture to escape from the area of the skin surrounding the wound. As is well-known to those of ordinary skill in the art, perforating the bandage will assist in preventing the skin from becoming macerated due to exposure to entrapped moisture.

Adhesive composition 30 is preferably any pressure sensitive biocompatible adhesive. Preferably, it is a water-based or solvent-based acrylate, rubber-based adhesive or a synthetic rubber-based adhesive, or a silicone adhesive. More preferably, it is a polyvinyl ester-based acrylic adhesive. The adhesives useful in the products of this invention can have relatively low aggressiveness, i.e., their adhesion-to-steel values can be relatively low in comparison with previously-used adhesives, but they will have sufficient adhesion to the skin when used in combination with the backing materials of this invention. For example, adhesion-to-steel values may be from about 45 g/cm to about 134 g/cm (about 4 oz/in to about 12 oz/in). The adhesives useful in the products of this invention may also be those used in traditional bandages, but at lower coating-weights, i.e., from about 20 g/m² to about 40 g/m², more preferably from about 25 to about 35 g/m² and most preferably, about 30 g/m².

Absorbent pad 40 is preferably a laminated or layered pad containing a wound-release surface, and an absorbent body. Preferably, the wound-release surface, designed to permit easy removal from a healing wound without disturbing the natural healing process or opening the wound again, is made from a polyethylene netting material or the like, known to those of ordinary skill in the art. Preferably, the absorbent body of absorbent pad 40 is a woven or non-woven batt made from fibers selected from the group of natural or synthetic absorbent and/or heat-sealable fibrous material such as cotton, rayon, polypropylene-blends or the like. Most preferably, the absorbent body of absorbent pad 40 is made from a 90% polypropylene, 10% rayon blend which can be heat-laminated with the wound-release surface. The absorbent body may be laminated to the wound-release surface using radiant or ultrasonic energy, or they may be folded together so as to expose the wound-release surface to the side of the bandage which contacts the skin of the wearer. Figures 2 and 3 represent additional embodiments of the disposable bandages of this invention, indicating that they can be made in different shapes and sizes including, but not limited to those set forth in Figures 2 and 3.

Preferably, the products of this invention are sufficiently conformable to adhere comfortably to the skin of the wearer and remain removably attached without aggressive adhesive attachment. However, they should be sufficiently resilient to retain their shape and return to their approximate original conformation when an applied stress from skin or body movement is removed. They need to conform easily to the body, within the range of forces generated by body and skin movement, and be able to withstand the forces they are subjected to when applied to the joints, yet they should not move or exert excessive pressure themselves.

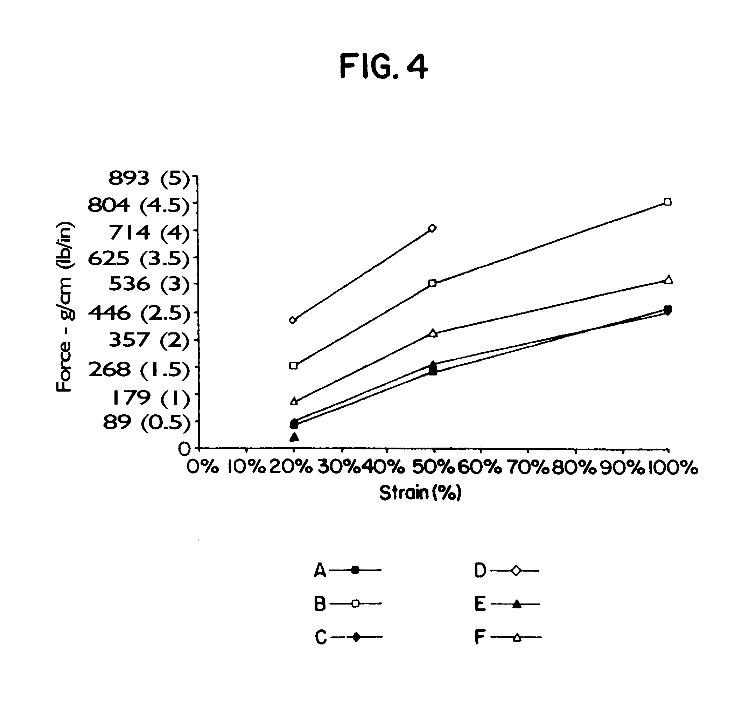

The properties which are desirable in the backings and products of this invention can be measured in terms of the force (in pounds per inch) needed to subject a bandage of this invention to a given strain, and the energy recovered upon relaxation of the product. Tensile strength at the breaking point and elongation are also useful measures of the products of this invention. Data obtained from tests performed in accordance with the following procedures are illustrated in Figures 4-7.

### Force vs. Strain

Force in relation to strain can be measured as follows: the products of this invention are conditioned according to TAPPI standards for four hours at 21°C (70°F) and 65% relative humidity. The products are placed in the clamps of an Instron testing machine at a gauge length of 6.35 cm (2.5"). The products are then stretched sequentially at a rate of 12.7 cm (5 inches) per minute to 20%, 50% and 100% strain. The force required to stretch the bandage products is measured at each value and normalized to the width of the sample. The force required for a 20% strain should be about 36 to 125 g/cm (about 0.2 to about 0.7 lb/in) and, for 50% strain, about 214 to 321 g/cm (about 1.2 to about 1.8 lb/in).

### Recovered Energy

Recovered energy, or the ratio of load to unload energy, is another important characteristic of the products of this invention. This parameter measures the amount of energy recovered upon relaxation of the bandage from different strains. The value of this parameter as measured in the products of this invention should be relatively high, so as to assure that the bandage will not permanently deform in use. Preferably, the recovered energy should be about 60 % to about 70% at 20% strain and about 40% to about 50% at 50% strain. The bandage should retain a relatively large amount of energy so as to provide an acceptable amount of resiliency in use.

### Tensile Breaking Strength

The tensile breaking strength of the products of this invention is another useful measurement of the conformability requirements of the products of this invention. The tensile breaking strength may be measured as follows: a continually increasing load is applied longitudinally to a specimen and the test is carried to rupture. Values for the breaking load are observed from machine scales or dials or recording charts. The samples are conditioned under TAPPI requirements at 23°C and 50% relative humidity for four hours. The specimen is held between two clamps in an Instron testing machine and strained by a uniform movement of the pulling clamp. The initial gauge length is 5.1 cm (2"). The pulling clamp has a speed of 51cm (20 inches) per minute for products having a breaking time of 10 seconds. If the breaking time is less than 10 seconds, the pulling speed is to be modified until the breaking time is 10 seconds. The average breaking force of the products of this invention should be between about 0.625 kg/cm and about 1.07 kg/cm (about 3.5 and about 6 pounds per inch). Preferably, it should be between about 0.71 kg/cm and about 0.89 kg/cm (about 4 and about 5 pounds/in).

### Maximum Elongation

Maximum elongation is another measure of conformability. The products of this invention should be quite conformable and, therefore, exhibit a high percent elongation without breaking. Maximum elongation may be measured using the same apparatus as set forth above in the breaking strength test. However, rather than measuring the force required to break the product, the length of the elongated product at the point of breakage is measured. The products of this invention should have be maximum elongation of at least about 150% and, preferably at least about 200% of the original length of the product. This ensures that the product will conform to the topography of the skin as it moves and shifts in wear.

The products of this invention should be made in such a way as to preserve the conformability and strength of the backing material during manufacture. Disposable bandage products are generally made using materials in rollstock. Preferably, the bandages of this invention are assembled such that the backing material is isolated from the other elements of the final product so as to preserve the properties of the backing material. This can be accomplished by running it over a roll, such as a vacuum drum, which accelerates the backing material before it reaches the place at which the absorbent pad is adhered to the backing material. This permits a certain amount of "slack" between the feeding of the backing material and the feeding of the absorbent pad, preserving the elastic nature of the backing material without stretching it. Thus, the backing material should be relaxed prior to assembly by running it slightly faster than the rate at which the remaining elements of the products are run. The adhesive is preferably applied to the backing material by transfer coating, although it may be wiped or applied using a doctor blade. The absorbent pad, which has been, preferably, formed into a laminate, is then adhered to the backing material by means of the adhesive. The product is then, preferably, perforated using hot pins. The process for making the products of this invention is described in EP-A-0676283 which is hereby incorporated by reference herein.

The products of this invention may be used by placing the disposable bandage of this invention over a wound or irritation, pressing the adhesive surface of the backing material into firm engagement with the skin of the wearer. Should the wound be open, the absorbent pad will absorb the wound exudate and prevent it from being carrier further onto the skin and/or clothing of the wearer. Furthermore, medicament may be placed directly in the pad prior to placement on the skin.

### Example 1

Disposable bandages were made in accordance with the method of this invention. The backing material was a closed-cell plasticized polyvinyl chloride foam, having a density of 0.48 g/cm³ (30 lb/ft³) and a thickness of 0.5mm (20 mils). The backing material was available coated with an acrylic adhesive, Avery EF 3508 available from the Avery Specialty Tape Division, Avery Dennison Corporation of Painesville, Ohio, was coated onto the foam at a weight of about 30 g/m². An absorbent pad composed of a 90% polypropylene, 10% rayon blend was laminated on one side to a clear polyethylene netting. Strip-style bandages were prepared from a roll of continuous backing material having a width equal to the overall length of the desired bandage. The absorbent pad material had a width equal to the desired pad area. The backing material was fed by a vacuum drum where the moving velocity of the backing material was increased slightly. Downstream of the vacuum drum, the velocity of the backing material was then slowed so that the elastic nature of the material was preserved. The absorbent pad material was then applied to the relaxed base material. The absorbent pad material was then perforated using hot pins. Facing tabs were placed on the exposed areas of the disposable bandage to protect the exposed adhesive prior to placement on the skin of a wearer. The facing tabs were made from 18kg (40 lb)/ream bleached draft paper coated with 5.9 kg (13 lb)/ream of polyethylene and Akrosil G9C0 silicone release coating on one side, although they may be made of any releasable material known to those of skill in the art.

### Example 2

The disposable bandages made in accordance with the method set forth in Example 1 were tested to determine whether they would stay in place on the skin of a wearer in accordance with the following method. Six disposable bandages of this invention were placed on the first and second knuckle of the fingers of each hand and on the dorsal surface of the upper arms of twenty-four human subjects. There were no restrictions on the normal activities or bathing habits of the subjects. The subjects wore the bandages for twenty-four hours. At the end of twenty-four hours, the subjects were examined and it was determined that the bandages of this invention remained intact and adhered to the subjects in an acceptable manner.

### Example 3

Bandages made in accordance with the method set forth in Example 1 were subjected to maximum force vs strain, load energy vs. strain, recovered energy, tensile breaking strength and percent elongation tests. Other products were also tested. Products A and C were products of this invention made in accordance with the method of Example 1 from plasticized polyvinyl chloride foam. The backing material of Product A and C had a density of 0.48 g/cm³ (30 lb/ft³) and a thickness of 0.5mm (20 mils). The backing material of Product B was a polyvinyl chloride foam which had a density of 0.48 g/cm³ (30 lb/ft³) and a thickness of 0.5mm (20 mils), but was stiffer than the foams used in Products A and C. Product D was a Band-Aid® brand Sheer Strip bandage available from Johnson & Johnson Consumer Products, Inc. of Skillman, New Jersey, which has a vinyl backing material. Product E was a Band-Aid® brand Flexible Fabric bandage available from Johnson & Johnson Consumer Products, Inc. of Skillman, New Jersey, which has a woven fabric backing material. Product F was an Active Strip Flexible Foam Bandage available from 3M of Minneapolis, Minnesota, which has a polyvinyl chloride foam backing.

The tests were conducted in accordance with the methods set forth above. The results of the maximum force vs. strain test are set forth in Figure 4. The tests indicate that Products A and C had fairly low relative values of maximum force per percent strain. This indicates that the force required to bend or deform the bandage was relatively low, compared to the other bandages. This is important in determining whether the bandage is acceptably deformable for use in accordance with this invention.

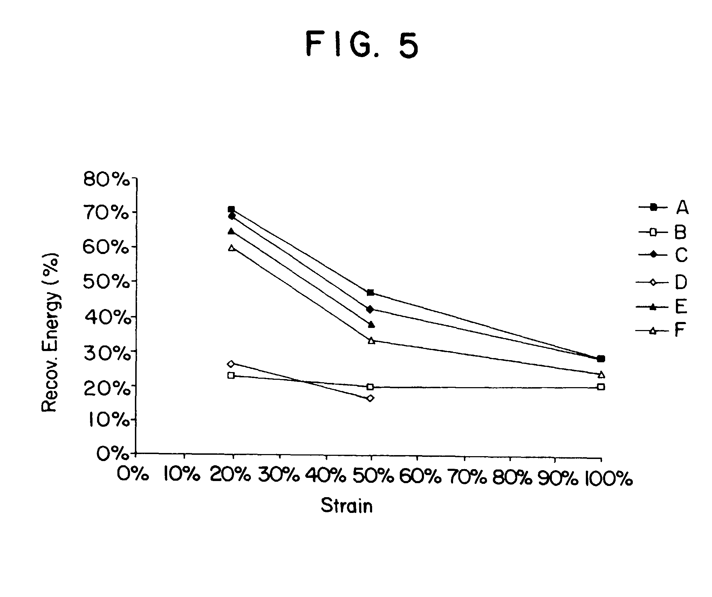

The results of the recovered energy test are set forth in Figure 5. This test measures the amount of energy recovered upon removal of a force subjecting the bandage to a given strain. The results of this test show that, particularly at lower strains, the recovered energy is high for Products A and C, and remains high in relation to the other tested products, even at 100% strain.

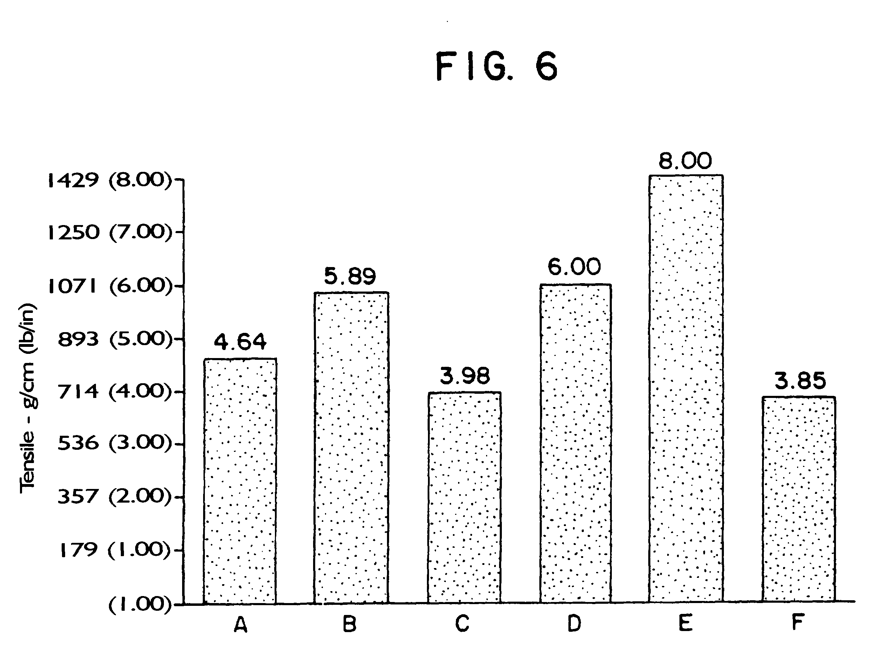

Tensile breaking strength was tested in accordance with the procedure set forth above. The results of this test are set forth in Figure 6. The tensile breaking strength of the products of this invention are generally between about 0.625 kg/cm and 0.89 kg/cm (about 3.5 and 5 pounds/in). Thus, the backing material of these products might break when subjected to the forces of the manufacturing process if it is not relaxed or supported during the process. This process permits the use of backing material which has a relatively low breaking strength.

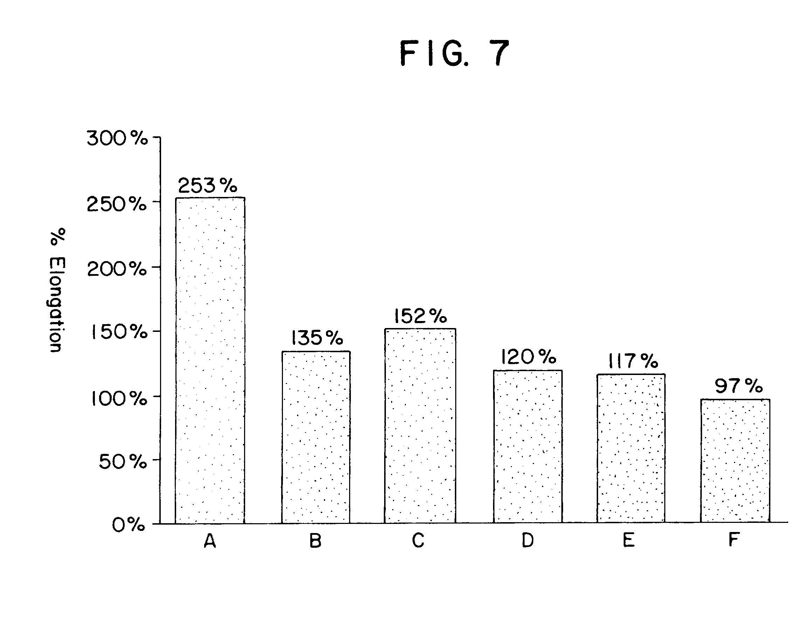

Percent elongation of Products A-F was also tested. The results are set forth in Figure 7. The products of this invention exhibit higher elongations than any of the other products, e.g., 253% and 152%. It should be noted that the elongation testing was performed using a pulling speed of 51cm (20 inches)/minute except for that in the case of Product E, in which case the pulling speed was 30.5cm (12 inches)/minute. The high percent elongation of the products of this invention indicate the high degree of conformability evident in their performance.

From the foregoing description of the products of the invention, others can devise alternate and equivalent constructions which are within the scope of the invention, as defined in the claims. Thus, the products of this invention are not limited to those specifically described herein.

## Claims

1. A disposable adhesive bandage comprising a backing material (20), an adhesive layer (30) and an absorbent pad (40), characterized in that said bandage requires a force of 36 to 125 g/cm (0.2 to 0.7 lb/in) to produce a 20% strain, and a force of 214 to 321 g/cm (1.2 to 1.8 lb/in) to produce a 50% strain.

2. A bandage according to claim 1 wherein said bandage exhibits a recovered energy of 60 to 70% at 20% strain, and a recovered energy of 40 to 50% at 50% strain.

3. A bandage according to claim 1 or claim 2, wherein the backing material (20) comprises a polymeric film or a polymeric foam, said foam or film comprising a polymer selected from the group consisting of polyvinyl chloride, polyethylene and polyurethane.

4. A bandage according to claim 3, wherein the backing material (20) comprises a plasticized polyvinyl chloride foam.

5. A bandage according to claim 4, wherein the foam has a density of 0.4 to 0.56 g/cm³ (25 to 35 lbs/ft³).

6. A bandage according to claim 4 or claim 5, wherein the foam has a thickness of 0.38 to 0.63 mm (15 to 25 mils).

7. A bandage according to any preceding claim, wherein the backing material (20) is perforated.

8. A bandage according to any preceding claim, wherein the adhesive layer (30) comprises an adhesive selected from the group consisting of solvent-based acrylate, water-based acrylate, natural rubber and silicone.

9. A bandage according to any preceding claim, wherein the adhesive layer (30) is present on the backing material (20) at a weight of 20 to 40 g/m².

## Patentansprüche

1. Ein wegwerfbarer Klebverband mit einem Rückenmaterial (20), einer Klebschicht (30) und einer absorbierenden Einlage (40), dadurch gekennzeichnet, daß besagter Verband eine Kraft von 36 bis 125 g/cm (0,2 bis 0,7 lb/in) zum Erzeugen einer 20 %-igen Dehnung und eine Kraft von 214 bis 321 g/cm (1,2 bis 1,8 lb/in) zum Erzeugen einer 50 %-igen Dehnung erfordert.

2. Ein Verband nach Anspruch 1, dadurch gekennzeichnet, daß besagter Verband eine rückgewonnene Energie von 60 bis 70 % bei einer 20 %-igen Dehnung und eine rückgewonnene Energie von 40 bis 50 % bei einer 50 %-igen Dehnung vorweist.

3. Ein Verband nach Anspruch 1 oder Anspruch 2, dadurch gekennzeichnet, daß das Rükkenmaterial (20) eine polymere Folie oder einen polymeren Schaum umfaßt, wobei besagter Schaum oder besagte Folie einen Polymer umfaßt, der aus der Gruppe mit Polyvinylchlorid, Polyethylen und Polyurethan ausgewählt ist.

4. Ein Verband nach Anspruch 3, dadurch gekennzeichnet, daß das Rückenmaterial (20) einen Weich-Polyvinychlorid-Schaum umfaßt.

5. Ein Verband nach Anspruch 4, dadurch gekennzeichnet, daß der Schaum eine Dichte von 0,4 bis 0,56 g/cm³ (25 bis 35 Ibs/ft³) aufweist.

6. Ein Verband nach Anspruch 4 oder Anspruch 5, dadurch gekennzeichnet, daß der Schaum eine Dicke von 0,38 bis 0,63 mm (15 bis 25 mils) aufweist.

7. Ein Verband nach irgendeinem vorangehenden Anspruch, dadurch gekennzeichnet, daß das Rückenmaterial (20) perforiert ist.

8. Ein Verband nach irgendeinem vorangehenden Anspruch, dadurch gekennzeichnet, daß die Klebschicht (30) einen Klebstoff umfaßt, der aus der Gruppe mit Acrylat auf Lösungsmittelbasis, Acrylat auf Wasserbasis, Naturgummi und Silikon ausgewählt ist.

9. Ein Verband nach irgendeinem vorangehenden Anspruch, dadurch gekennzeichnet, daß die Klebschicht (30) auf dem Rückenmaterial (20) mit einem Gewicht von 20 bis 40 g/m² vorhanden ist.

## Revendications

1. Bandage adhésif jetable comprenant un matériau support (20), une couche adhésive (30) et une compresse absorbante (40), caractérisé en ce que ledit bandage demande une force de 36 g/cm à 125 g/cm (de 0,2 lb/in à 0,7 lb/in) pour produire une déformation de 20 %, et une force de 214 g/cm à 321 g/cm (de 1,2 lb/in à 1,8 lb/in) pour produire une déformation de 50 %.

2. Bandage selon la revendication 1 où ledit bandage présente une énergie récupérée de 60 % à 70 % à une déformation de 20 %, et une énergie récupérée de 40 % à 50 % à une déformation de 50 %.

3. Bandage selon la revendication 1 ou 2, dans lequel le matériau support (20) comprend un film polymère ou une mousse polymère, ladite mousse ou ledit film comprenant un polymère sélectionné parmi le groupe se composant de chlorure de polyvinyle, de polyéthylène et de polyuréthane.

4. Bandage selon la revendication 3, dans lequel le matériau support (20) comprend une mousse plastifiée de chlorure de polyvinyle.

5. Bandage selon la revendication 4, dans lequel la mousse a une densité de 0,4 g/cm³ à 0,56 g/cm³ (de 25 lbs/ft³ à 35 lbs/ft³) .

6. Bandage selon la revendication 4 ou 5, dans lequel la mousse a une épaisseur comprise entre 0,38 mm et 0,63 mm (entre 15 mils et 25 mils).

7. Bandage selon l'une quelconque des revendications précédentes, dans lequel le matériau support (20) est perforé.

8. Bandage selon l'une quelconque des revendications précédentes, dans lequel la couche adhésive (30) comprend un adhésif sélectionné parmi le groupe se composant d'acrylate à base de solvant, d'acrylate à base d'eau, de caoutchouc naturel et de silicone.

9. Bandage selon l'une quelconque des revendications précédentes, dans lequel la couche adhésive (30) est présente sur le matériau support (20) suivant un poids de 20 g/m² à 40 g/m².
